# EUROPEAN PATENT APPLICATION

(11) **EP 4 186 679 A1**
(43) Date of publication of application: **31.05.2023**
(21) Application number: 22209070.6
(22) Date of filing: 23.11.2022
(51) Int. Cl.: B29C 48/00, B29C 48/09, B29C 48/11, B29C 48/32, B29D 23/00, B29C 48/25

(54) **EXTRUSION HEAD FOR CONTINUOUS EXTRUSION OF A MULTILUMEN MEDICAL TUBE, METHOD OF MANUFACTURING A MULTILUMEN TUBE AND A MEDICAL TUBE MANUFACTURED BY THIS METHOD**

(30) Priority: 24.11.2021 PL 43962821
(71) Applicant: Extrudan Sp. z o.o., 62-070 Dopiewo (PL)
(72) Inventor: Pedersen, John-Eric, 61-854 Poznan (PL)
(74) Representative: Kondrat, Mariusz

(57) **Abstract**

The invention relates to an extrusion head for the continuous extrusion of a multilumen medical tube, consisting of a monolithic sleeve and a mandrel embedded therein, characterised in that the mandrel consists of a first part **(4a)** and a second part **(4b),** where the first part **(4a)** of the mandrel is provided with an external thread and with fitter openings arranged circumferentially, in which the needles **(5)** are fixed, inside the first part **(4a)** of the mandrel an air supply channel is located, at the end of which there is a threaded hole for screwing in a fastening screw **(6)** immobilising the needles **(5),** the second part **(4b)** of the mandrel is provided with an internal thread matching the external thread of the first part **(4a)** of the mandrel. The invention also relates to a method for manufacturing a multilumen medical tube, characterised in that the head according to invention is mounted to the extruder, then a plastic is loaded into an extruder, mixed and pushed through the extruder screw while it is heated, then said plastic is extruded through the gap formed between the monolithic sleeve **(3)** and the mandrel assuming the shape of said gap, then the formed tube is successively pulled through the extruder, guided through the cooling bath with coolant and cut to a pre-set length. The invention also relates to a multilumen disposable medical tube with reduced weight, characterised by a diameter of 1.3 to 12 mm

## Description

The object of the invention is an extrusion head for continuous extrusion of a multilumen medical tube, a method for producing a multilumen medical tube and a medical tube produced by this method.

In the disposable medical products sector, the most important characteristics are chemical and biological non-reactivity, sterility and reliability. Further, the functionality of the product for a given application must be considered. For disposable products, ecology is also an important aspect in production and disposal. In this context, hollow-wall tubing offers an alternative to conventional tubing.

Application US2005260374 discloses a multi-lumen tube produced by extrusion. Wherein, the extrusion method includes extrusion through a head together with a specially designed mandrel. Wherein, the disclosed head includes an extrusion tip having an outer surface; an extrusion nozzle surrounding said tip and having an inner surface cooperating with said tip surface to define a flow channel therebetween, said flow channel determining the direction of flow of said molten polymer in said head; and at least one clearance pipe assembly passing through the wall of said die into said flow channel, said pipe assembly having a first part extending transversely to said flow direction and a second part extending parallel to said flow direction, said first and second pipe parts joining at right angles. The shape of the cross-sectional clearance can be easily controlled by selecting a suitable end shape for the pipe clearance and properly placing the pipe radially inside the die by means of its pin vise.

Application US2001027819 discloses an extruder nozzle for use with an extrusion device having an extrusion chamber, the extruder nozzle being adapted to form, a shaped opening through which extrudable material is conveyed to form a tube having a length and shaped cross section. The nozzle comprises a portion of the die opening containing a flat sheet of rigid material attached to the extrusion chamber and having an opening; a mandrel slidably mounted in the extrusion chamber and adapted to move alternately in said direction of flow, said mandrel comprising an elongated rod having a clearance-forming portion at its lower end, at least a portion of said clearance-forming portion extending into the interior of said die opening and partially blocking the flow of extrudable material through said die opening, wherein said clearance forming portion comprises a first portion having a first cross-sectional shape and a second portion having a cross-sectional shape that differs from, said first cross-sectional shape; and means for reciprocally moving said mandrel to position either said first portion or said second portion of said clearance forming portion in said die opening.

The aim of the invention is to develop means and a method for producing a modified disposable medical tube to increase patient and healthcare personnel comfort and reduce the amount of waste generated.

The subject of invention is an extrusion head for the continuous extrusion of a multilumen medical tube comprising a monolithic sleeve and a mandrel embedded therein, characterised in that the mandrel comprises a first part and a second part, wherein the first part of the mandrel is provided with an external thread and welded holes arranged circumferentially, in which needles are fixed, inside the first part of the mandrel an air supply channel is located, at the end of which there is a threaded hole for screwing in a fastening screw immobilising the needles, the second part of the mandrel is provided with an internal thread matching the external thread of the first part of the mandrel.

Preferably, the head contains between 6 and 24 needles.

Preferably, the second part of the mandrel, in longitudinal section is essentially trapezoidal in shape.

Preferably, the screw has a tapered opening.

Another subject of the invention is a method of manufacturing a multilumen medical tube, characterised in that the head according to the invention is mounted to an extruder, a plastic is then loaded into the extruder, mixed and pushed through the extruder screw while being heated, and then said plastic is extruded through the gap formed between the monolithic sleeve and the mandrel assuming the shape of said gap, the formed tube is then successively pulled through the extruder, guided through the cooling bath with coolant and cut to a predetermined length.

Preferably, the plastic loaded into the extruder is selected from the group comprising polyvinyl chloride, thermoplastic elastomers, polyethylene.

Another subject of the invention is a multi-lumen disposable medical tube with reduced weight, characterised in that its diameter ranges from, 1.3 to 12 mm

The invention provides the following benefits:
- ensures a reduction in the amount of material used in production, while maintaining the appropriate mechanical properties of the conductor, which serves ecological purposes: reduction of waste, reduction of material consumption in production (and thus a lower overall demand for material whose production is not ecological);
- ensures that a tube with reduced weight is obtained, and the weight reduction also has a major impact on reducing the product's carbon footprint: from reduced production at the material manufacturer, to each subsequent transport stage (lighter packages, reduced fuel consumption), etc.
- provides a medical tube with reduced weight and lighter tubes for increased comfort for medical staff and patients.

The invention is shown in exemplary embodiments in the drawing, in which fig. 1 presents the overview of the head according to the invention; fig. 2 presents the head according to the invention in front view and in longitudinal section A-A; fig. 3 presents a longitudinal cross-sectional view of the mandrel of the head according to the invention in an configuration unfolded to the first part including the screw and to the second part; fig. 4 presents a longitudinal section of the first part of the mandrel of the head according to the invention together with the screw; fig. 5 presents an overview, cross-section and in longitudinal section A-A of the medical tube manufactured by the method according to the invention.

### Example 1.

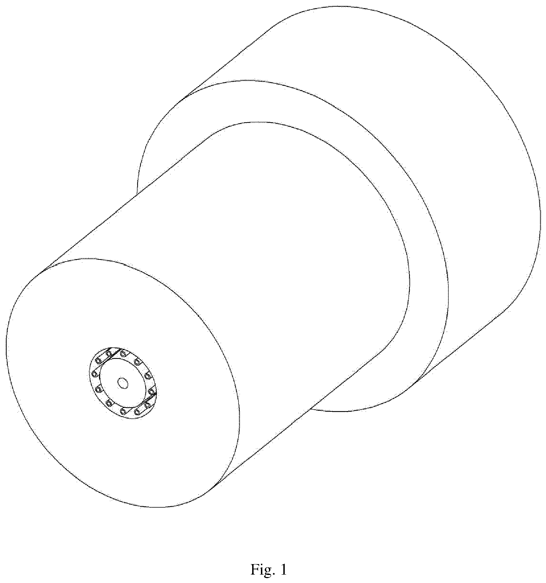

The head according to the invention is shown in figs. 1-4, wherein **3** stands for the monolithic sleeve, **4a** stands for the first part of the mandrel, **4b** stands for the second part of the mandrel, **5** stands for the needles, **6** stands for the fastening screw, **6.1** stands for the tapered opening, **7** stands for the air supply channel.

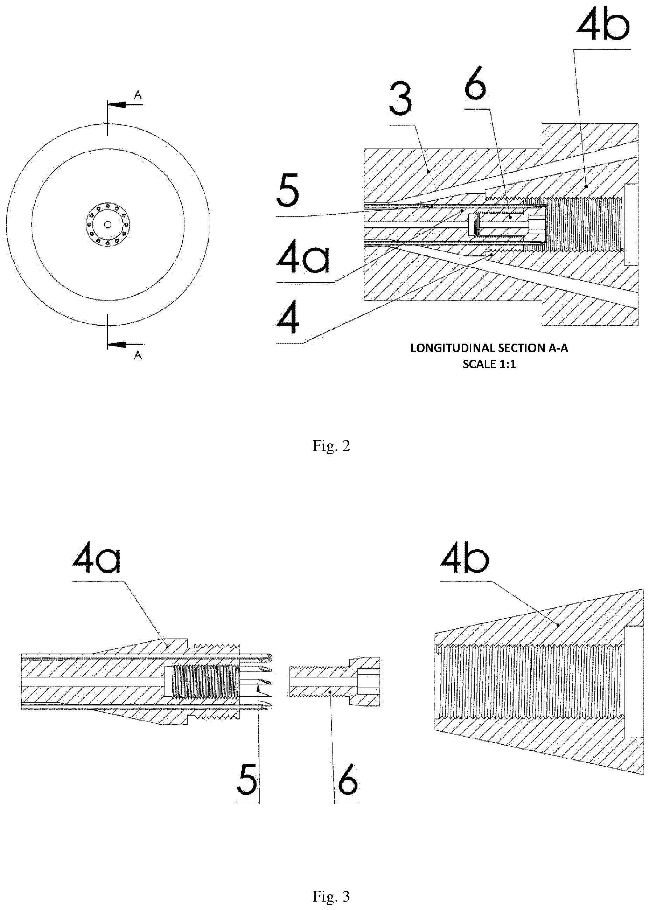

As indicated in figs. 1-2, the extrusion head for continuous extrusion of a multilumen medical tube according to the invention consists of the monolithic sleeve **3** and the mandrel embedded therein comprising the first part **4a** and the second part **4b**. The first part **4a** of the mandrel is provided with external thread and with fitted holes arranged circumferentially, in which needles **5** are fixed (figs. 3-4). Furthermore, inside the first part **4a** of the mandrel an air supply channel **7** is located, at the end of which there is a threaded hole for screwing in a fastening screw **6** immobilising the needles **5**. In contrast, the second part **4b** of the mandrel is provided with an internal thread matching the external thread of the first part **4a** of the mandrel. Furthermore, in this exemplary embodiment, the second part **4b** of the mandrel is substantially trapezoidal in longitudinal section (fig. 3). Whereby, the air channel in the screw **6** is an extension of the air supply channel **7** in the first part **4a** of the mandrel and serves to supply air into the "hollow" spaces of the fabricated tube **1** (i.e. into both air voids **2** and the interior of the tube **1** itself), otherwise the atmospheric pressure would "crush" the tube **1**.

The first part **4a** of the mandrel can be easily unscrewed from the second part **4b** of the mandrel gaining access to the needles **5** and the fixing screw **6**.

In this exemplary embodiment, the head according to the invention is provided with twelve needles **5**. Wherein, these are commercially available injection needles, cheap, accessible and of fixed dimensions. The fastening screw **6**, on the other hand, is a typical screw, but turned into a slight cone so that it slightly deflects the needles **5** during tightening without permanent deformation.

This slight bending is sufficient to prevent the needles **5** from moving during the extrusion process by the method according to the invention. The holes for the needles **5** are fitted in such a way that they can be inserted and removed by hand, but no extruded material enters the gap. At the same time, the fastening with screw **6** without permanent deformation of the needles **5** allows their removal without damaging the part of the mandrel **4a** even with deformation of the part of the needles forming the air voids **2** in the tubes.

Existing equipment solutions lack the ability to repair a sensitive production tool quickly and efficiently. This poses a huge problem, as a damaged monolithic mandrel from known solutions has to be returned to a specialist machining workshop.

On the other hand, the construction of the head according to the invention makes its eventual repair (the delicate needles **5** are easily damaged) and cleaning (the material tends to collect and burn in places where the flow is not sufficiently smooth, e.g. where the needles **5** attached, and these spaces usually cannot be cleaned properly without damaging the needles **5**) quick and inexpensive, which is important for mass production.

### Example 2.

Head as in example 1, except that it is fitted with six needles 5.

### Example 3.

Head as in example 1, except that it is fitted with twenty-four needles 5.

### Example 4.

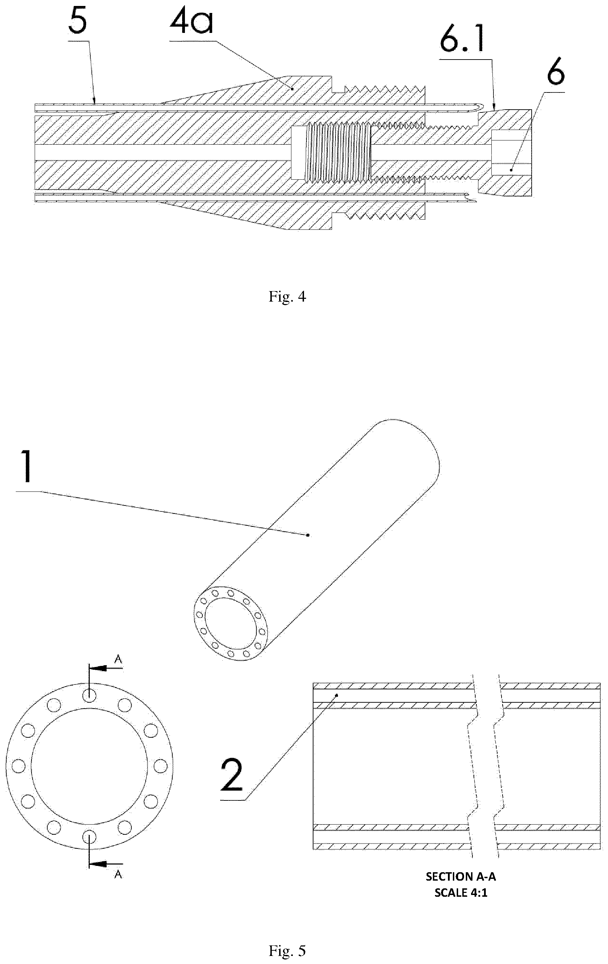

In this exemplary embodiment, a standard extrusion head kit with head mounted according to the invention of example 1 (fig. 1) was used to produce the multilumen medical tube 1 according the invention shown in fig. 5. In a first step, the plastic is loaded into the extruder, where it is mixed and pushed through the extruder screw while heating said plastic. Subsequently, said plastic is extruded through the gap formed between the monolithic sleeve **3** and the mandrel taking the shape of said gap. At the same time, the presence of the needles **5** ensures the formation of additional air voids **2**. The formed tube is then sequentially: pulled by an extruder, guided through a cooling bath with coolant (e.g. cold water) and cut to the desired length by an automatic cutter.

In this embodiment, the material for medical tube 1 was polyvinyl chloride (PVC), while other plastics such as thermoplastic elastomers (TPE) or polyethylene (PE) can be used to manufacture the tube.

Based on the method according to the invention, it is possible to obtain a flexible medical tube with reduced weight from the walls of which material has been removed obtaining voids (air voids **2**) with an outer diameter in the range from 1.3 mm to 12 mm. In this embodiment, the tube **1** with an internal diameter of 8.3 mm was obtained. Whereby, the outer diameter of the tube **1** depends on the size of the mandrel and the sleeve **3**, but the tube **1** is usually extracted (pulled by the extractor faster than it is extruded) so that an outer diameter of the tube **1** smaller than the hole in the sleeve **3** by about 70% can be obtained. Hence, a tube **1** of the same diameter can be obtained from, different sizes of mandrel and sleeve **3**.

### Example 5.

The weight-reduced multilumen disposable medical tube according to the invention can have an outer diameter of 1.3 mm to 12 mm. In this exemplary embodiment, the weight-reduced multilumen disposable medical tube according to the invention has an outer diameter of 8.3 mm, and has been manufactured in the manner of example 4 using the head of example 1. As indicated in fig. 5, the tube **1** has an inner diameter corresponding to the outer diameter similarly to conventional tubes, but with twelve air voids **2**. Furthermore, in this embodiment, the said tube is made of PVC. However, other plastics, such as TPE or PE, can be used for its manufacture. The tube was then compared with a medical hose known from the state of the art. Since, to the knowledge of the authors, medical multilumen tubes with an outer diameter equal to or less than 12 mm do not exist in the state of the art, a conventional hose with an outer diameter of 8.3 mm and a weight of 37 grams per metre was used as the reference product, which was manufactured from the same plastic (i.e. PVC) as the medical tube with air voids **2** according to the invention. In contrast, tube **1** according to the invention, with an outer diameter of 8.3 mm, showed a mass of 33.3 grams per metre and was therefore 10% lighter than the reference hose, while maintaining the same dimensions and all the required parameters in terms of strength, flexibility, reliability and chemical and biological reactivity. The smaller volume and weight dimensions of the tube **1** according to the invention provide a significant reduction in the amount of waste generated (which is particularly important in the case of single-use plastic medical products, which are a toxic waste to be incinerated) and in the energy input for transporting lower-weight parcels. An additional advantage is the increased comfort of patients and medical staff using the tube **1** according to the invention.

## Claims

1. An extrusion head for the continuous extrusion of a multilumen medical tube, consisting of a monolithic sleeve and a mandrel embedded therein, **characterised in that** the mandrel consists of a first part (**4a**) and a second part (**4b**), where the first part (**4a**) of the mandrel is provided with an external thread and with fitter openings arranged circumferentially, in which needles (**5**) are fixed, inside the first part (**4a**) of the mandrel an air supply channel is located, at the end of which there is a threaded hole for screwing in a fastening screw (**6**) immobilising the needles (**5**), the second part (**4b**) of the mandrel is provided with an internal thread matching the external thread of the first part (**4a**) of the mandrel.

2. The head according to claim. 1, **characterised in that** it contains from 6 to 24 needles (**5**).

3. The head according to claim. 1 or 2, **characterised in that** the second part (**4b**) of the mandrel in longitudinal section is essentially trapezoidal in shape.

4. The head according to any of the preceding claims. 1 to 3, **characterised in that** the screw (**6**) has a tapered opening (**6.1**).

5. A method for manufacturing a multilumen medical tube, **characterised in that** the head according to any of the preceding claims 1 to 4 is mounted to the extruder, then a plastic is loaded into an extruder, mixed and pushed through the extruder screw while it is heated, then said plastic is extruded through the gap formed between the monolithic sleeve (**3**) and the mandrel assuming the shape of said gap, then the formed tube is successively pulled through the extruder, guided through the cooling bath with coolant and cut to a pre-set length.

6. The method according to claim. 5, **characterised in that** the plastic loaded into the extruder is selected from the group comprising polyvinyl chloride, thermoplastic elastomers, polyethylene.

7. A multilumen disposable medical tube with reduced weight, **characterised by a** diameter of 1.3 to 12 mm
